# EUROPEAN PATENT SPECIFICATION

(11) **EP 4 498 915 B1**
(45) Date of publication and mention of the grant of the patent: **12.08.2026**
(21) Application number: 23711457.4
(22) Date of filing: 15.03.2023
(51) Int. Cl.: A61B 5/364, A61B 5/282, A61B 5/00

(54) **REPEATED INTERMITTENT ECG EVENT DETECTION**
WIEDERHOLTE INTERMITTIERENDE EKG-EREIGNISERKENNUNG
DÉTECTION D'ÉVÉNEMENT INTERMITTENT RÉPÉTÉ D'ECG

(30) Priority: 30.03.2022 US 202263325198 P
(43) Date of publication of application: 05.02.2025
(73) Proprietor: Koninklijke Philips N.V., 5656 AE Eindhoven (NL)
(72) Inventor: GREGG, Richard Earl, 5656 AG Eindhoven (NL)
(74) Representative: Philips Intellectual Property & Standards
(86) International application number: PCT/EP2023/056531
(87) International publication number: WO 2023/186533

(56) References cited:
- US-A1- 2004 127 805
- US-A1- 2007 073 176
- US-A1- 2019 090 774
- US-A1- 2021 338 135
- VESSELA KRASTEVA ET AL: "QRS Template Matching for Recognition of Ventricular Ectopic Beats", ANNALS OF BIOMEDICAL ENGINEERING, KLUWER ACADEMIC PUBLISHERS-PLENUM PUBLISHERS, NE, vol. 35, no. 12, 1 September 2007 (2007-09-01), pages 2065 - 2076, XP019547587, ISSN: 1573-9686, DOI: 10.1007/S10439-007-9368-9
- LI DAWEI ET AL: "Efficient Localization of Origins of PVC based on Random Signal Segmentation", 2021 IEEE 34TH INTERNATIONAL SYSTEM-ON-CHIP CONFERENCE (SOCC), IEEE, 14 September 2021 (2021-09-14), pages 141 - 145, XP034105671, DOI: 10.1109/SOCC52499.2021.9739421

## Description

### BACKGROUND

Frequent premature ventricular contractions (PVCs) have been identified as a likely cause of heart failure. Catheter ablation for resolving premature ventricular contractions is more effective than drug treatment. Ablation treatment for frequent premature ventricular contractions results in improved left ventricular ejection fraction, a measure of cardiac pumping performance. The ablation procedure can take a long time due to the time spent electrical-mapping the area to ablate in the left ventricle.

An origin of premature ventricular contraction for ablation can be found with reasonable accuracy using 12-lead electrocardiograph (ECG). The origin of premature ventricular contractions in left versus right outflow tracts can be improved with the addition of posterior electrocardiograph leads and additional electrocardiograph criteria.

US 2021/338135 A1 discloses a determination device which includes a data extracting unit for extracting ECG data over a predetermined period of time, an analysis unit for processing the ECG data extracted by the data extracting unit, and a determination model for producing origin information about the origin of the arrhythmia.

Reference is made to the following paper: Li Dawei et al: "Efficient localization of origins of PVC based on random signal segmentation", IEEE, 14 September 2021, pages 141-145. This teaches a method for localizing origin of a PVC based on random signal segmentation.

### SUMMARY

According to an aspect of the present invention, a controller includes a memory that stores instructions; and a processor that executes the instructions according to claims 1-6.

According to another aspect of the present invention, a computer program product is provided according to claims 7-11.

According to another aspect of the present invention, a system includes an electrocardiography apparatus and a display. The electrocardiography apparatus includes a memory that stores instructions and a processor that executes the instructions according to claim 12.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
FIG. 1A illustrates a system for repeated intermittent electrocardiograph event detection, in accordance with a representative embodiment.
FIG. 1B illustrates a device for repeated intermittent electrocardiograph event detection, in accordance with a representative embodiment.
FIG. 1C illustrates a controller for repeated intermittent electrocardiograph event detection, in accordance with a representative embodiment.
FIG. 2A illustrates a method for repeated intermittent electrocardiograph event detection.
FIG. 2B illustrates a method for repeated intermittent electrocardiograph event detection.
FIG. 3 illustrates a visualization from three leads in a 12-lead electrocardiograph report showing a premature ventricular contraction for repeated intermittent electrocardiograph event detection, in accordance with a representative embodiment.
FIG. 4 illustrates a visualization from all twelve leads in a 12-lead electrocardiograph report showing a premature ventricular contraction for repeated intermittent electrocardiograph event detection, in accordance with a representative embodiment.
FIG. 5 illustrates a computer system, on which a method for repeated intermittent electrocardiograph event detection is implemented, in accordance with another representative embodiment.

### DETAILED DESCRIPTION

In the following detailed description, for the purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms 'a', 'an' and 'the' are intended to include both singular and plural forms, unless the context clearly dictates otherwise. Additionally, the terms "comprises", and/or "comprising," and/or similar terms when used in this specification, specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to", "coupled to", or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as to not obscure the description of the example embodiments. Such methods and apparatuses are within the scope of the present disclosure.

As described herein, the shape of the premature ventricular contraction in electrocardiographic terms can be used to predict the origin of the premature ventricular contraction and therefore simplify the electrical mapping procedure to find the exact location for ablation. An electrocardiograph signal may be recorded and analyzed to predict premature ventricular contraction origin and provides helpful reports to aid in the ablation procedure planning.

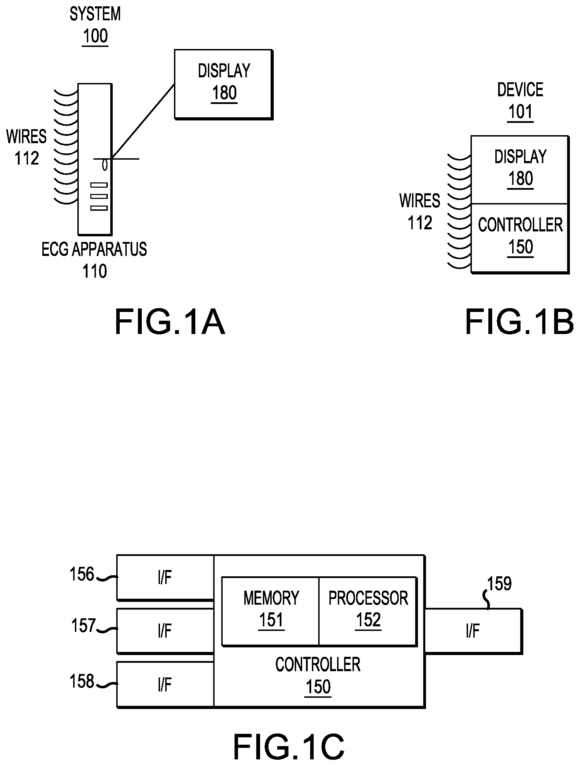

FIG. 1A illustrates a system 100 for repeated intermittent electrocardiograph event detection, in accordance with a representative embodiment.

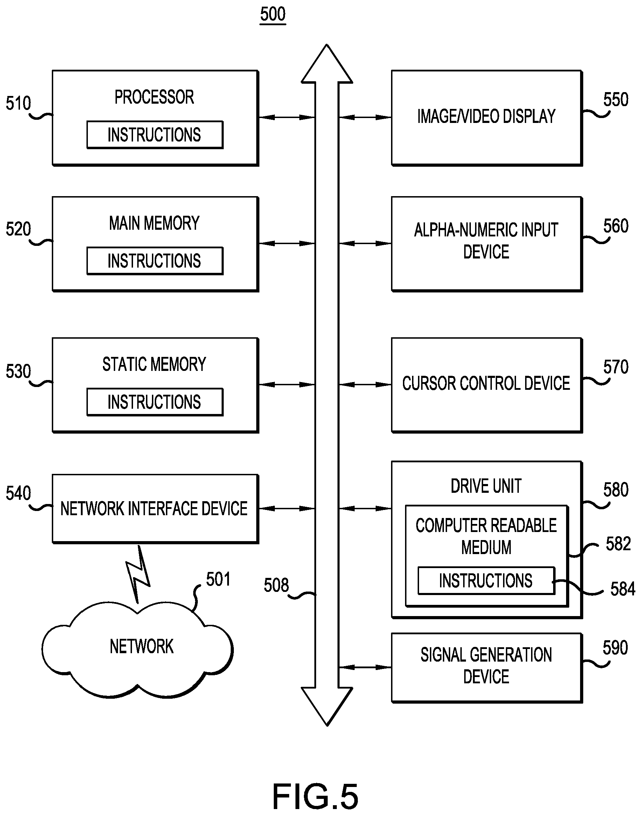

The system 100 in FIG. 1A is a system for repeated intermittent electrocardiograph event detection and includes components that may be provided together or that may be distributed. The system 100 includes an electrocardiograph (ECG) apparatus 110 with wires 112, and a display 180. A computer system that can be used to implement some of the logical circuitry of the ECG apparatus 110 is depicted in FIG. 5, though the ECG apparatus 110 may include more or fewer elements than depicted in FIG. 5. The ECG apparatus 110 may be a 12-lead electrocardiograph apparatus, adapted to implement the repeated intermittent electrocardiograph event detection described herein. The ECG apparatus 110 may be a device 101 as shown in FIG. 1B and may include a controller 150 as shown in and described with respect to FIG. 1C. The wires 112 may include ten wires in a 12-lead electrocardiograph configuration, with each wire terminating with an electrode, so as to include nine signal electrodes and one active ground electrode.

A 12-lead electrocardiograph apparatus uses nine signal electrodes and one active ground electrode. Three signal electrodes are placed on limbs including the right arm, left arm, and left leg. Six signal electrodes are placed on the chest. The active ground or reference electrode is placed on the right leg, although it may be placed anywhere. Each of the nine signal electrodes acts in combination with one or more other of the nine signal electrodes to detect voltages produced by depolarization and repolarization of the sum of individual heart muscle cells. The detected voltages are combined and processed to produce twelve tracings of time varying voltages as the 12 electrocardiograph leads. The 12 electrocardiograph leads consist of three limb leads (I, II, and III), three augmented limb leads (aVR, aVL, and aVF), and six precordial leads (V1 to V6). The three limb leads and the three augmented limb leads record the electrical potentials in the frontal plane. The six precordial leads V1-V6 are located on the torso of the patient. The twelve tracings of time varying voltages for an ECG apparatus 110 which has 12 leads are as listed in Table 1 below:

**TABLE 1**

| Lead | Voltage |
|---|---|
| I | VL - VR |
| II | VF - VR |
| III | VF - VL |
| aVR | VR - (VL + VF)/2 |
| aVL | VL - (VR + VF)/2 |
| aVF | VF - (VL + VR)/2 |
| V1 | V1 - (VR + VL+ VF)/3 |
| V2 | V2 - (VR + VL + VF)/3 |
| V3 | V3 - (VR + VL + VF)/3 |
| V4 | V4 - (VR + VL + VF)/3 |
| V5 | V5 - (VR + VL + VF)/3 |
| V6 | V6 - (VR + VL + VF)/3 |

In a standard electrocardiograph system for generating short term electrocardiographic recordings of supine subjects, the potentials indicated above, and their associated electrode positions, are: VL potential of an electrode on the left arm; VR potential of an electrode on the right arm; VF potential of an electrode on the left leg; V1 potential of an electrode on the front chest, right of sternum in the 4th rib interspace; V2 potential of an electrode on the front chest, left of sternum in the 4th rib interspace; V4 potential of an electrode at the left mid-clavicular line in the 5th rib interspace; V3 potential of an electrode midway between the V2 and V4 electrodes; V6 potential of an electrode at the left mid-axillary line in the 5th rib interspace; V5 potential of an electrode midway between the V4 and V6 electrodes; G (not indicated above) is an active ground or reference potential with respect to which potentials VL, VR, VF, and V1 through V6 are measured. As noted, the active ground or reference voltage is typically, though not necessarily, positioned on the right leg.

The ECG apparatus 110 may also include a user interface such as one or more buttons used to control the ECG apparatus 110. Such buttons may include a Start and/or Stop button, an On and/or Off button, and other types of user interface buttons used to control the operation of the ECG apparatus 110.

The display 180 may be local to the ECG apparatus 110 or may be remotely connected to the ECG apparatus 110. The display 180 may be connected to the ECG apparatus 110 via a local wired interface such as an HDMI cable or via a local wireless interface such as a Wi-Fi connection. The display 180 may be interfaced with other user input devices by which users can input instructions, including microphones for spoken instructions, mouses, keyboards, thumbwheels, gyro-accelerometer based gesture devices and so on.

The display 180 may be a monitor such as a computer monitor, a display on a mobile device, an augmented reality display, a television, an electronic whiteboard, or another screen configured to display electronic imagery. The display 180 may also include one or more input interface(s) such as those noted above that may connect other elements or components to the controller 150, as well as an interactive touch screen configured to display prompts to users and collect touch input from users.

FIG. 1B illustrates a device 101 for repeated intermittent electrocardiograph event detection, in accordance with a representative embodiment.

The device 101 includes a controller 150, the display 180 and the wires 112. An example of the device 101 is an electrocardiograph machine that includes a user interface for a user to interactively enter instructions to control the electrocardiograph machine so that results of an electrocardiograph recording are displayed on the display 180. The wires 112 may be used to implement the twelve leads of a 12-lead electrocardiograph machine.

A controller 150 is further depicted in FIG. 1C, and includes at least a memory 151 that stores instructions and a processor 152 that executes the instructions to implement some or all aspects of the methods described herein. A processor for the device 101 may be a single-core processor that executes instructions linearly, or a multicore processor that executes instructions in parallel using different cores. A computer that can be used to implement the controller 150 is depicted in FIG. 5, though a device 101 including the controller 150 may include more or fewer elements than depicted in FIG. 5.

The ECG apparatus 110 and the device 101 may each be connected to a printer to as to print periods of the electrocardiograph readings. The periods may be output to a printer and/or on the display 180, and my include all twelve leads of a 12-lead electrocardiograph.

FIG. 1C illustrates controller 150 for repeated intermittent electrocardiograph event detection, in accordance with a representative embodiment.

The controller 150 includes a memory 151, a processor 152, a first interface 156, a second interface 157, a third interface 158, and a fourth interface 159. The memory 151 stores instructions which are executed by the processor 152. The processor 152 executes the instructions.

The first interface 156, the second interface 157 and the third interface 158 may include ports, disk drives, wireless antennas, or other types of receiver circuitry. The fourth interface 159 may be a user interface, such as an interface by which a user may interactively enter instructions to control the controller 150 to implement some or all aspects of methods described herein.

The controller 150 may perform some of the operations described herein directly and may implement other operations described herein indirectly. For example, The controller 150 may indirectly control other operations such as by generating and transmitting content to be displayed on the display 180. Accordingly, the processes implemented by the controller 150 when the processor 152 executes instructions from the memory 151 may include steps not directly performed by the controller 150.

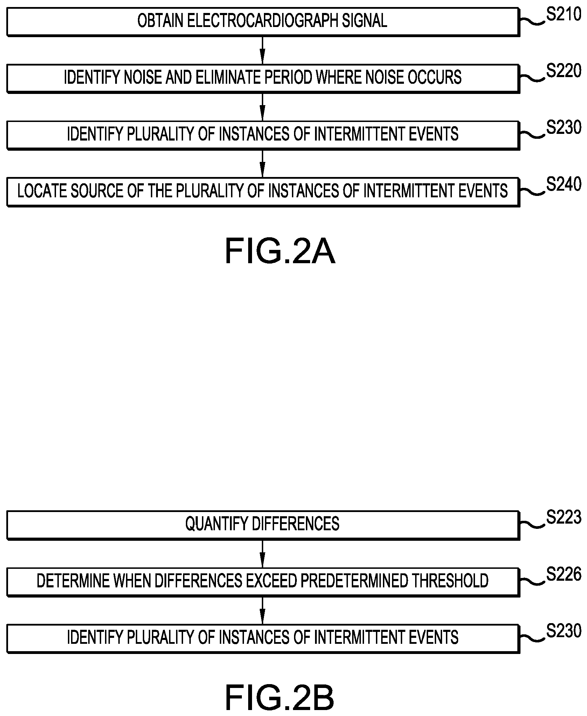

FIG. 2A illustrates a method for repeated intermittent electrocardiograph event detection.

The method of FIG. 2A starts by obtaining an electrocardiograph signal. The electrocardiograph signal may be obtained on an intermediate basis, such as for a period of 5 minutes or 10 minutes or 20 minutes, and may be obtained by the ECG apparatus 110 or the device 101. Electrocardiograph signal obtained in the method of FIG. 2A may be obtained by recording the electrocardiograph signal in real-time. The electrocardiograph signal may be a high resolution electrocardiograph signal. The recording for a length such as 5 minutes, 10 minutes or 20 minutes may capture many instances of each common premature ventricular contraction shape.

The electrocardiograph signal may be obtained at S210 by applying the ECG apparatus 110 or the device 101 to a patient with symptoms such as palpitations. The palpitations may be caused at a location in the left ventricle where tissue is irritated, and the irritated tissue generates signals that lead to the heart pumping even though the irritated tissue is not the proper natural source of signals to control the heart pumping. In the example of PVCs which are most often intermittent, the source is an estimate of the physical source in the patient's heart. Therefore, ablation of the irritated tissue may be an appropriate remedy to ensure that the heart is not pumping based on the signals from the irritated tissue at the source of the intermittent events identifiable in a full 12-lead electrocardiograph report. The ECG apparatus 110 or the device 101 may be applied to identify and visualize a premature ventricular complex corresponding to the anatomical source of the palpitations. If the symptoms are strong enough to warrant ablation, a cardiologist may use the repeated intermittent electrocardiograph event detection described herein to identify the source of the palpitations in the anatomy, so that the source may be ablated. Whereas a 10-second electrocardiograph report may only result in showing premature ventricular complex in just 3 of 12 leads with a narrow view of the heart, if at all, the full 12-lead electrocardiograph report from an intermediate-length electrocardiograph recording may be used to identify the source of palpitations in three-dimensional anatomy of the left ventricle of the patient.

At S220, the method of FIG. 2A includes identifying noise and eliminating a period where noise occurs. The period where noise occurs may be identified by analyzing data of the electrocardiograph signal, and identifying anomalies in the data so that a period in which the anomalies occurs may be eliminated from consideration in the event identification described herein. That is, since noisy periods may not result in trustworthy readings, the periods in which noise is identified may be discarded or otherwise disqualified from the analysis that follows at S230.

At S230, the method of FIG. 2A includes identifying a plurality of instances of intermittent events. The plurality of instances of intermittent events may be a cardiac event that occurs periodically or intermittently, and that are detectable in the same session in which the electrocardiograph signals are obtained at S210. The cardiac events that are detected may be arrhythmia events, for example. The plurality of instances of intermittent events may be identified in all twelve leads for a 12-lead electrocardiograph, and this may provide an ability to identify a source of palpitations. The 12 different leads of a 12-lead electrocardiograph provide multiple different angles to visualize the source of palpitations in the left ventricle, and the intermittent events that appear in the waveforms of the electrocardiograph signal can be identified and analyzed to identify the location of the source of the palpitations.

The identification at S230 may involve analyzing the recorded electrocardiograph signal with a real-time electrocardiograph algorithms applied by the ECG apparatus 110 or the device 101. As an example, a real-time Holter electrocardiograph algorithm may be applied to detect premature ventricular contractions and create templates of premature ventricular contractions. Each premature ventricular contraction template may be output from the Holter electrocardiograph algorithm and may include the count of how many premature ventricular contractions were included in each averaged beat template.

At S240, the method of FIG. 2A includes locating a source of the plurality of instances of intermittent events. For example, the controller 150 may apply a software algorithm to information of the plurality of instances of intermittent events to locate a location in the anatomy of the patient being subject to examination by the ECG apparatus 110 or the device 101 to locate the source of the plurality of instances of intermittent events. The location of the source may be displayed on the display 180 relative to other anatomical features of the patient. The location may be provided in a report along with data explaining the basis for identifying the location. The data may include times of the intermittent events, sources of the electrocardiograph signal(s) from which the intermittent events were identified, and characteristics of the electrocardiograph signals(s) from which the intermittent events were identified. Sources of the electrocardiograph signal(s) may be or include wires of the wires 112, or leads based on comparative signals from sets of two of the wires 112.

The locating at S240 may include analyzing premature ventricular contraction morphology to determine the most likely origin of the premature ventricular contractions in the left ventricle. The origin may be inside or outside the left ventricle. The premature ventricular contraction origin may be shown on a 17-segment bull's eye representation of the left ventricle.

The relative seriousness of the intermittent events may also be described, along with other information, generally the more frequent, the more serious. The location of the source of the plurality of instances of intermittent events may be identified during the same session in which the electrocardiograph signals are obtained at S210. Therefore, the location may be identified while the electrocardiograph signal is obtained in real-time or in near real-time. The method of FIG. 2A may include recording the electrocardiograph signal in real-time, so that location is identified and displayed on the display 180 while the electrocardiograph signal is obtained in real-time.

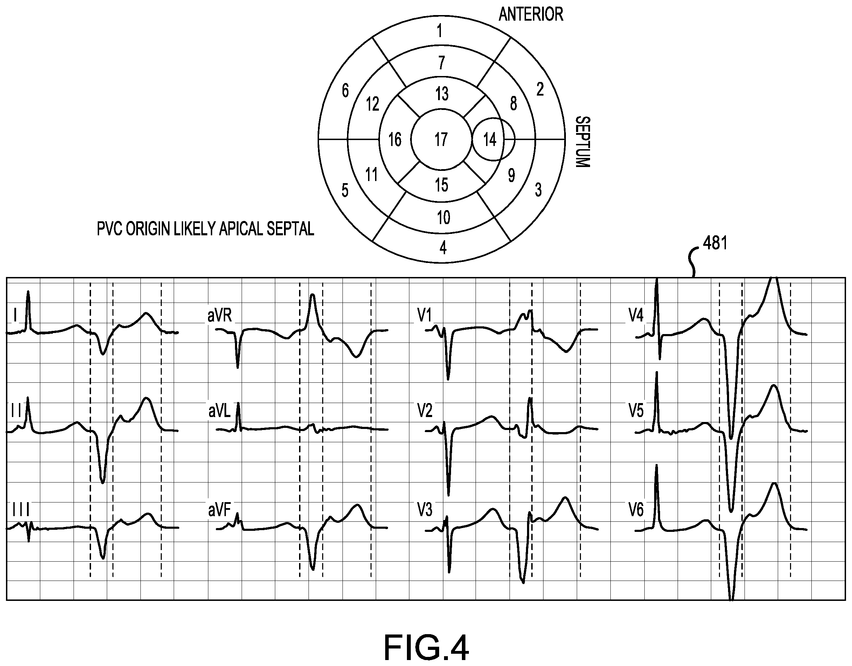

Locating the source of the premature ventricular contractions at S240 may be used to guide a surgical intervention to ablate the source. For example, a cardiologist may view all twelve leads of a 12-lead electrocardiograph report, and know where to roughly insert the catheter. A bulls-eye chart at the top of FIG. 4 may be displayed electronically, and the source region in the bulls-eye chart may be highlighted to confirm the location of the source in the surgical intervention.

FIG. 2B illustrates a method for repeated intermittent electrocardiograph event detection.

The method of FIG. 2B starts at S223 by quantifying differences. The differences which are quantified may be differences between characteristics of the electrocardiograph signal and characteristics of a baseline. The baseline may be a predetermined baseline or may be dynamically adjusted on an ongoing basis, such as a running average of measurements of the electrocardiograph signal. Many types of differences from electrocardiograph signals may be quantified at S223. For example, an individual waveform may have an amplitude, and adjacent waveforms may have frequencies and periods. Differences may be identified between minimums, maximums and/or medians, such as minimum amplitudes, maximum amplitudes and/or median amplitudes. Different PVCs may be compared to quantify differences. Similar PVCs indicate the same source while different PVC morphology indicates multiple different sources of the multifocal PVCs.

In some embodiments, a quantification at S223 may be between periods such as heart rates at different PVC times. For example, if a heart rate in a 10 second segment starting at 40 seconds into an electrocardiograph session is 70 beats per minute, and the heart rate increases to 90 beats per minute in a 10 second segment starting at 4 minutes, 20 seconds into the electrocardiograph session, the increase in heart rate may be quantified as a difference and that difference may result in a heart rate influenced change in morphology. Analysis at S223 may, for example, search for the largest increase or decrease between heart rates during the electrocardiograph session, or the largest increase or decrease adjusted for the difference in times when the measurements of the compared heart rates are taken.

In some embodiments, a quantification at S223 may be between a difference between an extreme measurement and an average of measurements. For example, the ECG apparatus 110 may periodically or constantly record an average of measurements such as heart rates, and then compare the average to each new measurement to identify extreme measurements. In some embodiments, the average may be maintained on a trailing basis, such as the trailing 15 or 30 seconds. Differences that are larger than a predetermined threshold may result in identification of a reportable event at S230 and initiation of generation of a report at S240.

In some embodiments, comparisons may be between real-time measurements and baselines such as values that are considered "normal" for a demographic group which includes the patient. In some embodiments, comparisons may be between real-time measurements and averages, such as averages for a demographic group which includes the patient or averages specific to measurements of the patient.

In some embodiments, the quantification may involve differences between a characteristic derived from the electrocardiography signal and a baseline. For example, an amplitude of a waveform at a precise point in time may be considered a characteristic of the waveform, whereas an average of amplitudes may be derived from such a characteristic.

Quantification may occur between different beats classified as PVCs by algorithm which are not pure PVCs but (1) premature atrial contractions (PACs) with aberrant conduction or (2) fusion beats which are a mixture of normal conduction and a PVC. In both cases, PAC or fusion, the beats should not be used to find the source of the PVCs.

At S226, the method of FIG. 2B includes determining when the differences exceed a predetermined threshold. Predetermined thresholds may be set for multiple different characteristics of electrocardiograph signals. Additionally, predetermined thresholds may be customized based on demographic characteristics of patients. Thresholds may also be adjusted, such as for trailing averages of the characteristics, so that a measurement that is more (or less) than a trailing average by 20%, for example, indicates a reportable event.

At S230, the method of FIG. 2B includes identifying the plurality of instances of the intermittent events. The plurality of instances of intermittent events may be identified based on the differences exceeding a predetermined threshold as determined at S226. The plurality of instances of the intermittent events may be displayed graphically on the display 180, described as information including data in a report, and used to generate a visualization of the source of the plurality of instances of intermittent events in the context of patient anatomy on the display 180.

In some embodiments, a trained artificial intelligence model may be applied to identify the plurality of instances of intermittent events at S230. For example, a trained artificial intelligence model may be trained to identify characteristics of electrocardiograph waveforms that indicate an illness, and the training may be used to identify patients with an illness even though the patients are not complaining about related symptoms. Large datasets of electrocardiograph waveforms with varying corresponding diagnoses may be used to train such artificial intelligence models, and inputs to such trained artificial intelligence models may include demographic information of patients as well as characteristics of the electrocardiograph waveforms.

Using the methods of FIG. 2A and FIG. 2B, multiple instances of intermittent events such as premature ventricular contraction may be captured and used to accurately locate the source of the premature ventricular contractions. This may be due, in part, to the fact that the electrocardiograph signals are captured for an intermediate length such as 5 minutes, 10 minutes or 20 minutes. In this regard, premature ventricular contractions are not consistently captured in shorter electrocardiograph snapshots such as 10 seconds, and longer monitoring electrocardiograph or Holter electrocardiograph recordings typically use fewer than twelve leads and therefore do not capture morphology well. Beat averaging by template reduces noise and allows for better localization of premature ventricular contraction source because presumably a noisy source signal results in a noisy localization estimate.

Additionally, the most frequent focus which produces premature ventricular contractions may be indicated based on the analysis in FIG. 2A and FIG. 2B. The most frequent focus is the heart location generating the most PVCs and therefore the most symptoms of palpitations.

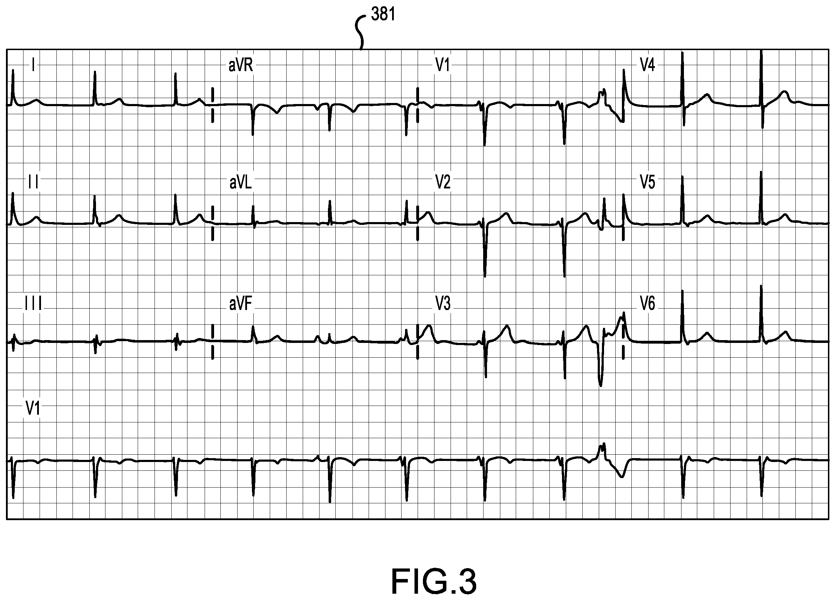

Additionally, the electrocardiograph signal obtained at S210 may be obtained via a short term recording in and office, in a clinic or in a hospital, as examples, and may provide results in real-time or near real-time without requiring a wait for an ambulatory record. Further, as reflected in FIG. 3 and as described below, conventional electrocardiograph reports do not show all 12-leads of premature ventricular contractions, and this makes visual expert validation of origin difficult. The locating of source in FIG. 2A and FIG. 2B is based on automated waveform analysis, and may consider all 12-leads. That is, a short-term recording of high-resolution electrocardiograph signal recordings may be analyzed in a office, clinic or hospital, for example, and the electrocardiograph analysis may detect premature ventricular contractions and average common premature ventricular contraction shapes in templates. Each premature ventricular contraction template may be displayed in a different 12-lead electrocardiograph style report so that all 12-leads are seen for each premature ventricular contraction template. The analysis of each premature ventricular contraction morphology/template is performed to determine the approximate left ventricle focus, or foci i.e. where to ablate to treat the premature ventricular contractions.

FIG. 3 illustrates a visualization from three leads in a 12-lead electrocardiograph report showing a premature ventricular contraction for repeated intermittent electrocardiograph event detection, in accordance with a representative embodiment.

The visualization in FIG. 3 may be a visualization displayed on a user interface 381, such as via the display 180 in FIG. 1A and FIG. 1B. In FIG. 3, the premature ventricular contraction appears in only three leads out of six chest leads and, therefore, the full morphology cannot be appreciated. electrocardiograph reports do not show all 12-leads of premature ventricular contractions, and this makes visual expert validation of origin difficult. Additionally, in FIG. 3, only a single premature ventricular contraction appears in the standard 12-lead report, whereas a premature ventricular contraction may be an intermittent event that can be better identified using the teachings herein.

The report for the premature ventricular contraction in FIG. 3 indicates a heart rate, data for a sinus rhythm, data for a ventricular premature complex, and other information. The top of the report may also identify demographic information of the patient, such as age and gender.

FIG. 4 illustrates a visualization from all twelve leads in a 12-lead electrocardiograph report showing a single premature ventricular contraction template for repeated intermittent electrocardiograph event detection, in accordance with a representative embodiment. There may be a separate display for each PVC template because each template may correspond to a different source location.

The visualization in FIG. 4 may be a visualization displayed on a user interface 481, such as via the display 180 in FIG. 1A and FIG. 1B. In FIG. 4, the same premature ventricular contraction as in FIG. 3 is now shown in all 12-leads so that the morphology can be fully appreciated. The first set of vertical lines mark the QRS complex. The last vertical line shows the end of the T-wave in the premature ventricular contraction heart cycle. The text above and 17-segment bull's eye indicates the premature ventricular contraction origin in the LV (i.e., in location 14, the apical septal segment).

A report may include the type of visualization on a user interface shown in FIG. 4, and may include each premature ventricular contraction template/morphology for review by a clinician.

Using the teachings herein, the origin of each premature ventricular contraction may be estimated, and the origin may be shown in a bulls-eye map (i.e., the 17-segment bull's eye) of the left ventricle. The premature ventricular contraction may be the focus of the analysis performed for repeated intermittent electrocardiograph event detection.

In some embodiments, a three-dimensional visualization may be used to identify the origin of palpitations. As an example, the bulls-eye map in FIG. 4 or an alternative three-dimensional visualization may be used to identify a region such as the septum, or the apex of the left ventricle.

FIG. 5 illustrates a computer system, on which a method for repeated intermittent electrocardiograph event detection is implemented, in accordance with another representative embodiment.

Referring to FIG.5, the computer system 500 includes a set of software instructions that can be executed to cause the computer system 500 to perform any of the methods or computer-based functions disclosed herein. The computer system 500 may operate as a standalone device or may be connected, for example, using a network 501, to other computer systems or peripheral devices. In embodiments, a computer system 500 performs logical processing based on digital signals received via an analog-to-digital converter.

In a networked deployment, the computer system 500 operates in the capacity of a server or as a client user computer in a server-client user network environment, or as a peer computer system in a peer-to-peer (or distributed) network environment. The computer system 500 can also be implemented as or incorporated into various devices, such as an ECG apparatus 110 or device 101 that includes a controller, a workstation, stationary computer, a mobile computer, a personal computer (PC), a laptop computer, a tablet computer, or any other machine capable of executing a set of software instructions (sequential or otherwise) that specify actions to be taken by that machine. The computer system 500 can be incorporated as or in a device that in turn is in an integrated system that includes additional devices. In an embodiment, the computer system 500 can be implemented using electronic devices that provide voice, video or data communication. Further, while the computer system 500 is illustrated in the singular, the term "system" shall also be taken to include any collection of systems or sub-systems that individually or jointly execute a set, or multiple sets, of software instructions to perform one or more computer functions.

As illustrated in FIG. 5, the computer system 500 includes a processor 510. The processor 510 may be considered a representative example of a processor of a controller and executes instructions to implement some or all aspects of methods and processes described herein. The processor 510 is tangible and non-transitory. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The processor 510 is an article of manufacture and/or a machine component. The processor 510 is configured to execute software instructions to perform functions as described in the various embodiments herein. The processor 510 may be a general-purpose processor or may be part of an application specific integrated circuit (ASIC). The processor 510 may also be a microprocessor, a microcomputer, a processor chip, a controller, a microcontroller, a digital signal processor (DSP), a state machine, or a programmable logic device. The processor 510 may also be a logical circuit, including a programmable gate array (PGA), such as a field programmable gate array (FPGA), or another type of circuit that includes discrete gate and/or transistor logic. The processor 510 may be a central processing unit (CPU), a graphics processing unit (GPU), or both. Additionally, any processor described herein may include multiple processors, parallel processors, or both. Multiple processors may be included in, or coupled to, a single device or multiple devices.

The term "processor" as used herein encompasses an electronic component able to execute a program or machine executable instruction. References to a computing device comprising "a processor" should be interpreted to include more than one processor or processing core, as in a multi-core processor. A processor may also refer to a collection of processors within a single computer system or distributed among multiple computer systems. The term computing device should also be interpreted to include a collection or network of computing devices each including a processor or processors. Programs have software instructions performed by one or multiple processors that may be within the same computing device or which may be distributed across multiple computing devices.

The computer system 500 further includes a main memory 520 and a static memory 530, where memories in the computer system 500 communicate with each other and the processor 510 via a bus 508. Either or both of the main memory 520 and the static memory 530 may be considered representative examples of a memory of a controller, and store instructions used to implement some or all aspects of methods and processes described herein. Memories described herein are tangible storage mediums for storing data and executable software instructions and are non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The main memory 520 and the static memory 530 are articles of manufacture and/or machine components. The main memory 520 and the static memory 530 are computer-readable mediums from which data and executable software instructions can be read by a computer (e.g., the processor 510). Each of the main memory 520 and the static memory 530 may be implemented as one or more of random access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM), digital versatile disk (DVD), floppy disk, blu-ray disk, or any other form of storage medium known in the art. The memories may be volatile or non-volatile, secure and/or encrypted, unsecure and/or unencrypted.

"Memory" is an example of a computer-readable storage medium. Computer memory is any memory which is directly accessible to a processor. Examples of computer memory include, but are not limited to RAM memory, registers, and register files. References to "computer memory" or "memory" should be interpreted as possibly being multiple memories. The memory may for instance be multiple memories within the same computer system. The memory may also be multiple memories distributed amongst multiple computer systems or computing devices.

As shown, the computer system 500 further includes a video display unit 550, such as a liquid crystal display (LCD), an organic light emitting diode (OLED), a flat panel display, a solid-state display, or a cathode ray tube (CRT), for example. Additionally, the computer system 500 includes an input device 560, such as a keyboard/virtual keyboard or touch-sensitive input screen or speech input with speech recognition, and a cursor control device 570, such as a mouse or touch-sensitive input screen or pad. The computer system 500 also optionally includes a disk drive unit 580, a signal generation device 590, such as a speaker or remote control, and/or a network interface device 540.

In an embodiment, as depicted in FIG. 5, the disk drive unit 580 includes a computer-readable medium 582 in which one or more sets of software instructions 584 (software) are embedded. The sets of software instructions 584 are read from the computer-readable medium 582 to be executed by the processor 510. Further, the software instructions 584, when executed by the processor 510, perform one or more steps of the methods and processes as described herein. In an embodiment, the software instructions 584 reside all or in part within the main memory 520, the static memory 530 and/or the processor 510 during execution by the computer system 500. Further, the computer-readable medium 582 may include software instructions 584 or receive and execute software instructions 584 responsive to a propagated signal, so that a device connected to a network 501 communicates voice, video or data over the network 501. The software instructions 584 may be transmitted or received over the network 501 via the network interface device 540.

In an embodiment, dedicated hardware implementations, such as application-specific integrated circuits (ASICs), field programmable gate arrays (FPGAs), programmable logic arrays and other hardware components, are constructed to implement one or more of the methods described herein. One or more embodiments described herein may implement functions using two or more specific interconnected hardware modules or devices with related control and data signals that can be communicated between and through the modules. Accordingly, the present disclosure encompasses software, firmware, and hardware implementations. Nothing in the present application should be interpreted as being implemented or implementable solely with software and not hardware such as a tangible non-transitory processor and/or memory.

In accordance with various embodiments of the present disclosure, the methods described herein may be implemented using a hardware computer system that executes software programs. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

## Claims

1. A controller (150), comprising:
a memory (151) that stores instructions; and
a processor (152) that executes the instructions, wherein, when executed by the processor, the instructions cause the controller to:
obtain a 12-lead electrocardiography signal;
identify a plurality of instances of intermittent events from the electrocardiography signal based on waveforms in the electrocardiography signal, wherein the intermittent events are premature ventricular contractions, PVCs;
generate a premature ventricular contraction, PVC, template from the plurality of instances of intermittent PVC events; and
locate a source of the plurality of instances of the intermittent events using the PVC template;
generate for a user interface display (180) at least one visualization of all 12 leads of the electrocardiography signal corresponding to the PVC template.

2. The controller of claim 1, wherein, when executed by the processor, the instructions cause the controller further to:
identify noise in the electrocardiography signal, and eliminate a period where the noise occurs.

3. The controller of either of claims 1 or 2, wherein, when executed by the processor, the instructions cause the controller further to:
quantify differences between the electrocardiography signal and a baseline;
determine when the differences exceed a predetermined threshold, and
identify the plurality of instances of the intermittent events based on determining that the differences exceed the predetermined threshold.

4. The controller of claim 3, wherein the differences comprise differences between characteristics of the electrocardiography signal and characteristics of the baseline.

5. The controller of claim 1, wherein, when executed by the processor, the instructions cause the controller further to:
quantify differences between a characteristic derived from the electrocardiography signal and a baseline;
determine when the differences exceed a predetermined threshold, and
identify the plurality of instances of the intermittent events based on determining that the differences exceed the predetermined threshold.

6. A computer program product, comprising instructions wherein the computer program, when executed by a processor (152), causes a computer apparatus to:
obtain a 12-lead electrocardiography signal;
identify a plurality of instances of intermittent events from the electrocardiography signal based on waveforms in the electrocardiography signal, wherein the intermittent events are premature ventricular contractions, PVCs,
generate a premature ventricular contraction, PVC, template from the plurality of instances of intermittent PVC events,
locate a source of the plurality of instances of the intermittent events using the PVC template,
generate for a user interface display (180) at least one visualization of all 12 leads of the electrocardiography signal corresponding to the PVC template.

7. The computer program product of claim 6 wherein the computer program, when executed by a processor, causes the computer apparatus further to: identify noise in the electrocardiography signal, and eliminate a period where the noise occurs.

8. The computer program product of claims 6 or 7 wherein the computer program, when executed by a processor, causes the computer apparatus further to:
quantify differences between the electrocardiography signal and a baseline;
determine when the differences exceed a predetermined threshold, and
identify the plurality of instances of the intermittent events based on determining that the differences exceed the predetermined threshold, wherein the differences comprise differences between characteristics of the electrocardiography signal and characteristics of the baseline.

9. A system (100), comprising:
an electrocardiography apparatus (110) including a memory (151) that stores instructions and a processor (152) that executes the instructions; and
a display (180),
wherein, when executed by the processor, the instructions cause the electrocardiography apparatus to:
obtain a 12-lead electrocardiography signal;
identify a plurality of instances of intermittent events from the electrocardiography signal based on waveforms in the electrocardiography signal, wherein the intermittent events are premature ventricular contractions, PVCs;
generate a premature ventricular contraction, PVC, template from the plurality of instances of intermittent PVC events;
locate a source of the plurality of instances of the intermittent events using the PVC template; and
initiate generation of a visualization of the source of the plurality of instances of the intermittent events on the display, and a visualization of all 12 leads of the electrocardiography signal corresponding to the PVC template.

## Patentansprüche

1. Steuereinheit (150), umfassend:
einen Speicher (151), der Anweisungen speichert; und
einen Prozessor (152), der die Anweisungen ausführt, wobei die Anweisungen, wenn vom Prozessor ausgeführt, die Steuereinheit zu Folgendem veranlassen:
Erhalten eines 12-Kanal-Elektrokardiographiesignals;
Identifizieren einer Vielzahl von Instanzen von intermittierenden Ereignissen aus dem Elektrokardiographiesignal anhand von Wellenformen in dem Elektrokardiographiesignal, wobei die intermittierenden Ereignissen vorzeitige ventrikuläre Kontraktionen (PVCs) sind;
Erzeugen einer Vorlage für eine vorzeitige ventrikuläre Kontraktion (PVC) aus der Vielzahl von Instanzen von intermittierenden PVC-Ereignissen; und
Ermitteln einer Quelle der Vielzahl von Instanzen der intermittierenden Ereignisse unter Verwendung der PVC-Vorlage;
Erzeugen mindestens einer Visualisierung aller 12 Kanäle des Elektrokardiographiesignals, die der PVC-Vorlage entsprechen, für eine Benutzeroberflächenanzeige (180).

2. Steuereinheit nach Anspruch 1, wobei die Anweisungen, wenn von dem Prozessor ausgeführt, die Steuereinheit weiter zu Folgendem veranlassen:
Identifizieren von Rauschen in dem Elektrokardiographiesignal und Eliminieren eines Zeitraums, in dem das Rauschen auftritt.

3. Steuereinheit nach einem der Ansprüche 1 oder 2, wobei die Anweisungen, wenn vom Prozessor ausgeführt, die Steuereinheit weiter zu Folgendem veranlassen:
Quantifizieren von Unterschieden zwischen dem Elektrokardiographiesignal und einer Grundlinie;
Bestimmen, wann die Unterschiede einen vorbestimmten Schwellenwert überschreiten, und
Identifizieren der Vielzahl von Instanzen der intermittierenden Ereignisse anhand des Bestimmens, dass die Unterschiede den vorbestimmten Schwellenwert überschreiten.

4. Steuereinheit nach Anspruch 3, wobei die Unterschiede Unterschiede zwischen Charakteristiken des Elektrokardiographiesignals und Charakteristiken der Grundlinie umfassen.

5. Steuereinheit nach Anspruch 1, wobei die Anweisungen, wenn vom Prozessor ausgeführt, die Steuereinheit weiter zu Folgendem veranlassen:
Quantifizieren von Unterschieden zwischen einer aus dem Elektrokardiographiesignal abgeleiteten Charakteristik und einer Grundlinie;
Bestimmen, wann die Unterschiede einen vorbestimmten Schwellenwert überschreiten, und
Identifizieren der Vielzahl von Instanzen der intermittierenden Ereignisse anhand des Bestimmens, dass die Unterschiede den vorbestimmten Schwellenwert überschreiten.

6. Computerprogrammprodukt, umfassend Anweisungen, wobei das Computerprogramm, wenn von einem Prozessor (152) ausgeführt, eine Computereinrichtung zu Folgendem veranlasst:
Erhalten eines 12-Kanal-Elektrokardiographiesignals;
Identifizieren einer Vielzahl von Instanzen von intermittierenden Ereignissen aus dem Elektrokardiographiesignal anhand von Wellenformen in dem Elektrokardiographiesignal, wobei die intermittierenden Ereignissen vorzeitige ventrikuläre Kontraktionen (PVCs) sind,
Erzeugen einer Vorlage für eine vorzeitige ventrikuläre Kontraktion (PVC) aus der Vielzahl von Instanzen von intermittierenden PVC-Ereignissen,
Ermitteln einer Quelle der Vielzahl von Instanzen der intermittierenden Ereignisse unter Verwendung der PVC-Vorlage,
Erzeugen mindestens einer Visualisierung aller 12 Kanäle des Elektrokardiographiesignals, die der PVC-Vorlage entsprechen, für eine Benutzeroberflächenanzeige (180).

7. Computerprogrammprodukt nach Anspruch 6, wobei das Computerprogramm, wenn von einem Prozessor ausgeführt, die Computereinrichtung zu Folgendem veranlasst:
Identifizieren von Rauschen in dem Elektrokardiographiesignal und Eliminieren eines Zeitraums, in dem das Rauschen auftritt.

8. Computerprogrammprodukt nach Ansprüchen 6 oder 7, wobei das Computerprogramm, wenn von einem Prozessor ausgeführt, die Computereinrichtung zu Folgendem veranlasst:
Quantifizieren von Unterschieden zwischen dem Elektrokardiographiesignal und einer Grundlinie;
Bestimmen, wann die Unterschiede einen vorbestimmten Schwellenwert überschreiten, und
Identifizieren der Vielzahl von Instanzen der intermittierender Ereignisse anhand von Bestimmen, dass die Unterschiede den vorbestimmten Schwellenwert überschreiten, wobei die Unterschiede Unterschiede zwischen Charakteristiken des Elektrokardiographiesignals und Charakteristiken der Grundlinie umfassen.

9. System (100), umfassend:
eine Elektrokardiographieeinrichtung (110), die einen Speicher (151), der Anweisungen speichert, und einem Prozessor (152) einschließt, der die Anweisungen ausführt; und
eine Anzeige (180),
wobei die Anweisungen, wenn vom Prozessor ausgeführt, die Elektrokardiographieeinrichtung zu Folgendem veranlassen:
Erhalten eines 12-Kanal-Elektrokardiographiesignals;
Identifizieren einer Vielzahl von Instanzen von intermittierenden Ereignissen aus dem Elektrokardiographiesignal anhand von Wellenformen in dem Elektrokardiographiesignal, wobei die intermittierenden Ereignissen vorzeitige ventrikuläre Kontraktionen (PVCs) sind;
Erzeugen einer Vorlage für eine vorzeitige ventrikuläre Kontraktion (PVC) aus der Vielzahl von Instanzen von intermittierenden PVC-Ereignissen;
Ermitteln einer Quelle der Vielzahl von Instanzen der intermittierenden Ereignisse unter Verwendung der PVC-Vorlage; und
Initiieren der Erzeugung einer Visualisierung der Quelle der Vielzahl von Instanzen der intermittierenden Ereignisse auf der Anzeige und einer Visualisierung aller 12 Kanäle des Elektrokardiographiesignals, die der PVC-Vorlage entsprechen.

## Revendications

1. Dispositif de commande (150), comprenant :
une mémoire (151) qui stocke des instructions ; et
un processeur (152) qui exécute les instructions, dans lequel, lorsqu'elles sont exécutées par le processeur, les instructions amènent le dispositif de commande à :
obtenir un signal électrocardiographique à 12 dérivations ;
identifier une pluralité d'événements intermittents à partir du signal électrocardiographique en fonction des formes d'onde du signal électrocardiographique, dans lequel les événements intermittents sont des contractions ventriculaires prématurées, PVC ;
générer un modèle de contraction ventriculaire prématurée (PVC) à partir de la pluralité d'événements de PVC intermittents ; et
localiser la source de la pluralité des occurrences des événements intermittents à l'aide du modèle de PVC ;
générer pour un affichage d'interface utilisateur (180) au moins une visualisation des 12 dérivations du signal d'électrocardiographie correspondant au modèle de PVC.

2. Dispositif de commande selon la revendication 1, dans lequel, lorsqu'elles sont exécutées par le processeur, les instructions amènent en outre le dispositif de commande à :
identifier le bruit dans le signal électrocardiographique et éliminer la période à laquelle ce bruit apparaît.

3. Dispositif de commande selon l'une ou l'autre des revendications 1 ou 2, dans lequel, lorsqu'elles sont exécutées par le processeur, les instructions amènent en outre le dispositif de commande à :
quantifier les différences entre le signal électrocardiographique et une ligne de base ;
déterminer à quel moment les différences dépassent un seuil prédéterminé, et
identifier la pluralité d'occurrences des événements intermittents en déterminant que les différences dépassent le seuil prédéterminé.

4. Dispositif de commande selon la revendication 3, dans lequel les différences comprennent des différences entre les caractéristiques du signal électrocardiographique et les caractéristiques de la ligne de base.

5. Dispositif de commande selon la revendication 1, dans lequel, lorsqu'elles sont exécutées par le processeur, les instructions amènent en outre le dispositif de commande à :
quantifier les différences entre une caractéristique dérivée du signal électrocardiographique et une ligne de base ;
déterminer à quel moment les différences dépassent un seuil prédéterminé, et
identifier la pluralité d'occurrences des événements intermittents en déterminant que les différences dépassent le seuil prédéterminé.

6. Produit de programme informatique, comprenant des instructions dans lequel le programme informatique, lorsqu'il est exécuté par un processeur (152), amène un appareil informatique à :
obtenir un signal électrocardiographique à 12 dérivations ;
identifier une pluralité d'événements intermittents à partir du signal électrocardiographique en se basant sur les formes d'onde du signal électrocardiographique, dans lequel les événements intermittents sont des contractions ventriculaires prématurées, PVC,
générer un modèle de contraction ventriculaire prématurée (PVC) à partir de la pluralité d'événements de PVC intermittents,
localiser la source de la pluralité des occurrences des événements intermittents à l'aide du modèle de PVC,
générer pour un affichage d'interface utilisateur (180) au moins une visualisation des 12 dérivations du signal d'électrocardiographie correspondant au modèle de PVC.

7. Produit de programme informatique selon la revendication 6, dans lequel le programme informatique, lorsqu'il est exécuté par un processeur, amène l'appareil informatique à :
identifier le bruit dans le signal électrocardiographique et éliminer la période à laquelle ce bruit apparaît.

8. Produit de programme informatique selon les revendications 6 ou 7, dans lequel le programme informatique, lorsqu'il est exécuté par un processeur, amène l'appareil informatique à :
quantifier les différences entre le signal électrocardiographique et une ligne de base ;
déterminer à quel moment les différences dépassent un seuil prédéterminé, et
identifier la pluralité des cas d'événements intermittents en déterminant que les différences dépassent le seuil prédéterminé, dans lequel les différences comprennent les différences entre les caractéristiques du signal électrocardiographique et les caractéristiques de la ligne de base.

9. Système (100), comprenant :
un appareil d'électrocardiographie (110) incluant une mémoire (151) qui stocke les instructions et un processeur (152) qui exécute les instructions ; et
un écran (180),
dans lequel, lorsqu'elles sont exécutées par le processeur, les instructions amènent l'appareil d'électrocardiographie à :
obtenir un signal électrocardiographique à 12 dérivations ;
identifier une pluralité d'événements intermittents à partir du signal électrocardiographique en fonction des formes d'onde du signal électrocardiographique, dans lequel les événements intermittents sont des contractions ventriculaires prématurées, PVC ;
générer un modèle de contraction ventriculaire prématurée, PVC, à partir de la pluralité d'événements de PVC intermittents ;
localiser la source de la pluralité d'occurrences des événements intermittents à l'aide du modèle de PVC ; et
lancer la génération d'une visualisation de la source de la pluralité d'occurrences des événements intermittents sur l'écran, et une visualisation de l'ensemble des 12 dérivations du signal électrocardiographique correspondant au modèle de PVC.
